(19)

Europäisches
Patentamt
European
Patent Office
Office européen
des brevets

(11) **EP 3 039 416 B1**

(12) **EUROPEAN PATENT SPECIFICATION**

(45) Date of publication and mention
of the grant of the patent:
**04.05.2022  Bulletin 2022/18**

(21) Application number: **14839658.3**

(22) Date of filing: **28.08.2014**

(51) International Patent Classification (IPC):
***G01N 27/447*** *(2006.01)*     ***B03C 5/00*** *(2006.01)*
***B03C 5/02*** *(2006.01)*

(52) Cooperative Patent Classification (CPC):
**B03C 5/005; B03C 5/026; G01N 30/0005;**
**G01N 33/4833;** B03C 2201/26; G01N 2030/0065

(86) International application number:
**PCT/US2014/053107**

(87) International publication number:
**WO 2015/031586 (05.03.2015 Gazette 2015/09)**

(54) **METHOD AND APPARATUS FOR ISOLATION, CAPTURE AND MOLECULAR ANALYSIS OF TARGET PARTICLES**

VERFAHREN UND VORRICHTUNG ZUR ISOLIERUNG, ERFASSUNG UND MOLEKULAREN ANALYSE VON ZIELPARTIKELN

PROCÉDÉ ET APPAREIL POUR L'ISOLEMENT, LA CAPTURE ET L'ANALYSE MOLÉCULAIRE DE PARTICULES CIBLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **29.08.2013  US 201361871624 P**

(43) Date of publication of application:
**06.07.2016  Bulletin 2016/27**

(73) Proprietor: **Precision for Medicine (TX), Inc.**
**Houston, TX 77054 (US)**

(72) Inventors:
• **MENACHERY, Anoop**
  **04229 Leipzig (DE)**
• **GUPTA, Vishal**
  **Pearland, TX 77584 (US)**

• **HASEGAWA, David, K.**
  **Cupertino, CA 95014 (US)**
• **PETHIG, Ronald**
  **Menai Bridge, Anglesey LL59 5DT (GB)**

(74) Representative: **Witte, Weller & Partner**
**Patentanwälte mbB**
**Postfach 10 54 62**
**70047 Stuttgart (DE)**

(56) References cited:
**WO-A1-2013/028573**   **WO-A2-2006/003214**
**US-A- 5 814 200**   **US-A- 5 993 631**
**US-A1- 2009 092 989**   **US-A1- 2011 139 620**
**US-A1- 2012 031 759**   **US-A1- 2012 048 736**
**US-A1- 2013 063 861**   **US-B1- 7 029 564**
**US-B1- 7 029 564**   **US-B2- 7 105 081**
**US-B2- 7 773 363**

**Description**

BACKGROUND OF THE INVENTION

The present invention relates generally to methods and apparatuses for separation and isolation of target particles. More particularly, the present invention is directed to new device elements, apparatuses employing the device elements, and methods for providing improved efficient separation and isolation of one or more types of target particles. The methods employ integrated fluidic devices using dielectrophoresis that are designed to perform a continuous, semi-continuous, staged (interval or intermittent), or sporadic, processing of micro fluids. The devices and apparatuses employ a chamber(s) capable of enriching and isolating target particles from a suspension and, thereby enable quantitatively or qualitatively, analyzing the enriched and isolated target particles.

More than 11 million people are diagnosed with cancer each year and it is estimated that there will be 16 million new cases every year by 2020 (Cho, Mol Cancer 2007; 6:25). Traditionally, pathologists have played a major role in the initial diagnosis of cancer, and in the morphologic classification and evaluation of the responsiveness of the patient to therapy, based upon analysis of tissue samples (e.g., serial biopsies).

CROSS-REFERENCE TO RELATED APPLICATIONS

In certain cancers, such as the various types of breast cancers, monitoring a patient's response to treatment is an essential component of therapy, since the degree of response can provide important prognostic information related to disease-free and overall survival. Histopathology provides an accurate assessment of treatment efficacy on the basis of the extent of residual tumor and regressive changes within the tumor tissue. However, only 20% of breast cancer patients achieve a pathologic complete response, a fact that demands better methods for monitoring therapeutic effectiveness particularly in the early stages of therapy (Avril, N. et al., The Journal of Nuclear Medicine, 50 (5) Suppl., May 2009, 55S-63S). Early identification is critical to survival and, early identification of ineffective therapy would also be very useful in patients suffering from metastatic breast and other types of cancer due to the number of palliative treatment options. New methods for predicting therapeutic effectiveness prior to and over the course of therapeutic treatment of various cancers, especially methods that are rapid, minimally invasive and available at an early stage of treatment, can help to individualize and guide treatment, avoid and/or minimize ineffective or unproductive chemotherapies, provide near real-time analyses, and allow early detection in patients at risk for early relapse.

Understanding the molecular determinants of cancer disease and therapy response has grown and resulted in various approaches to enable individualized patient treatment. However, current methods of examining the disease of an individual patient are primarily based on tissue samples (e.g., CT-guided biopsies or surgical material). These methods have several limitations that include invasive, risky collection procedures for the patient, limited accessibility of the tumor tissue, dependence upon the cancer type and location, and the use of patient's archival tumor samples that may have been collected several years prior to metastatic relapse, making the molecular information about the patients' tumor outdated. The combination of these factors results in less effective therapy for the patient and an unbeneficial economic impact to society. Furthermore, because of the nature of metastatic cancer, oncologists often have to wait several months before they can determine if a specific treatment is beneficial to the patient.

Therefore, better instruments need to be developed to provide and reach the goals of oncology, and further aid in saving lives by way of, for example, supporting early detection of cancer, real-time monitoring, and providing physicians with the ability to tailor patient-specific therapy to improve cancer patient survival.

More recently, there has been a significant advancement of our understanding of the molecular origins of different types of cancer and characteristics of tumor aggressiveness, based upon a major expansion of genomic and proteomic data. Cancer cells display a broad spectrum of genetic alterations that include gene rearrangements, point mutations and gene amplifications, which lead to disturbances in molecular pathways regulating cell growth, survival and metastasis. When such changes manifest themselves in patients (from a small percentage to a majority of patients) having a cancerous tumor, or receiving treatment with a chemotherapeutic agent having a particular mechanism of action, discovery and quantification of these changes can be used to identify biomarkers for detecting and developing targeted therapies, and for predicting the clinical response to chemotherapeutic drugs used to treat the disease. The identification of new predictive biomarkers can provide invaluable assistance to clinicians in minimally-invasively and rapidly predicting a patient's response to therapy, selecting the best treatment modality, monitoring response to treatment over the course of therapy, as well as post-therapy, to thereby improve the likelihood of overall and recurrence-free survival. The advantages of the above cannot be understated.

Molecular profiling can have special importance for predicting and monitoring response to cancer therapy. Major therapeutic developments have been observed in tumors with druggable targets. These include the use of KIT kinase inhibitors in KIT mutation-positive gastrointestinal stromal tumors (GIST) and ABL kinase inhibitors in BCR-ABL-positive chronic myelogenous leukemia (CML). Common solid tumors, such as breast, lung, and colorectal cancer have been difficult to treat, perhaps in part because they are heterogeneous, with each subset of patients having different molecular abnormalities, and sometimes within the tumor itself. Identifying relevant molecular subtypes within heterogeneous diseases, and matching patients with appropriate targeted agents or combinations of them is crucial to future therapeutic progress. For instance, patients with non-small-cell lung cancer (NSCLC) have a relatively modest response rate (RR) of 10% to EGFR tyrosine kinase inhibitors; however, the RR exceeds 70% in patients with an underlying EGFR mutation. Similarly, patients with NSCLC and an EML4-ALK fusion are very likely to benefit from PF-02341066, which is an ALK inhibitor. In other histologies, patients with melanoma and an underlying BRAF mutation demonstrated an unprecedented RR of 60% in a phase I clinical trial with the BRAF inhibitor PLX4032. Patients with medullary thyroid cancer and a RET mutation demonstrated durable responses with RET inhibitors in a phase I setting, exceeding treatment outcomes from the prior standard therapy.

These observations suggest that molecular profiling has special importance for cancer therapy. Mutation analysis is usually performed on archived tumor tissue or on tissue from fresh biopsies, which can be a limiting factor for its use. A sufficient amount of tissue for analysis is not available for about 25%-45% patients. Therefore, development of new techniques that would allow oncologists to obtain the most amount of information from the least amount of biologic material available is of paramount importance in the further development of personalized cancer therapy.

Circulating Tumor Cells (CTCs) are cancer cells that have disseminated from the primary cancer site and carry disease-relevant molecular information that can be exploited for molecular diagnostic applications. CTCs present in the blood stream at low frequency (as low as 1 cell in ~10 million peripheral blood mononuclear cells), offer a minimally invasive, "liquid biopsy" to assess a patient's tumor in real-time and enable profiling of a patient's tumor so that oncologists can match the right drug with the right patient to improve treatment and overall response outcomes. Molecular CTC information is needed by the physician to provide the most effective therapy to the patient. An adequate number of CTCs is needed in order to perform the molecular based assays to profile a patient's tumor.

Recent technologies have allowed the detection and isolation of CTCs. Historically, the detection and capture of such cells has been challenging (Gupta, et al., Biomicrofluidics 6, 024133 (2012)). Techniques currently used for CTC capture include immunomagnetic separation (Cohen, S.J., et al., J. Clin. Oncol. 26, 3213 (2008); Maheswaran, S., et al., N. Engl. J. Med. 359, 366 (2008), membrane filters (Desitter, I., et al., Anticancer Res. 31, 427 (2011), micro-electro-mechanical system chips (Nagrath, S., et al., Nature 450, 1235 (2007)), and dielectrophoretic field-flow fractionation (DEP-FFF) technology (Gupta, V., et al, ibid.).

Generally, CTC detection methods are composed of the following two steps: an enrichment (isolation) process and detection (identification) process (cytometric and nucleic acid techniques), which may or may not be separate from enrichment. Genetic and molecular characterization of CTCs is typically conducted by fluorescent in situ hybridization (FISH), comparative genomic hybridization (CGH), PCR-based techniques, and biomarker immunofluorescent staining. Normally absent from the peripheral blood of a healthy donor, CTC counts have been described to correlate negatively with progression-free survival and overall survival in patients with metastatic, colorectal, breast, and prostate cancer (Budd, G.T., Cristofanilli, M., Ellis, M.J., Stopeck, A., Borden, E., Miller, M.C., Matera, J., Repollet, M., Doyle, G.V., Terstappen, L.W., Hayes, D.F. (2006). Circulating tumor cells versus imaging--predicting overall survival in metastatic breast cancer. Clin Cancer Res 12(21), 6403-6409.

See also, De Bono, J.S., Scher, H.I., Montgomery, R.B., Parker, C., Miller, M.C., Tissing, H., Doyle, G.V., Terstappen, L.W., Pienta, K.J., Raghavan, D. (2008). Circulating tumor cells predict survival benefit from treatment in metastatic castration-resistant prostate cancer. Clin Cancer Res 14(19), 6302-6309; Cohen, S.J., Punt, C.J., Iannotti, N., Saidman, B.H., Sabbath, K.D., Gabrail, N.Y., Picus, J., Morse, M., Mitchell, E., Miller, M.C., Doyle, G.V., Tissing, H., Terstappen, L.W., Meropol, N.J. (2008), Relationship of circulating tumor cells to tumor response, progression-free survival, and overall survival in patients with metastatic colorectal cancer. J Clin Oncol 26(19), 3213-3221; and Cristofanilli, M., Budd, G.T., Ellis, M.J., Stopeck, A., Matera, J., Miller, M.C., Reuben, J.M., Doyle, G.V., Allard, W.J., Terstappen, L.W., Hayes, D.F. (2004). Circulating tumor cells, disease progression, and survival in metastatic breast cancer. N Engl J Med 351(8), 781-791.).

Despite the urgent need for alternative approaches, there is only one FDA cleared test (CELLSEARCH® by Veridex, LLC; Johnson & Johnson) on the commercial market that utilizes CTCs. The CELLSEARCH® test is an antibody-dependent method that has been approved by the FDA for only three cancer types. This method performs magnetic separation using a specific surface biomarker-namely an epithelial cell adhesion molecule known as EpCAM. However, not all cancer cells express this marker. Also, the CELLSEARCH® test does not provide the ability to generate any molecular information about the cancer. Furthermore, other CTC technologies are limited in their ability to efficiently capture an adequate number of CTCs from whole blood and from various types of cancer. Thus, currently available technologies do not solve the challenge of providing physicians and drug development companies conducting clinical trials in oncology with a broadly applicable, efficient, and economical method which allows fast analysis of predictive markers on CTCs for diagnosis, prognosis and therapy.

Accordingly, there remains a need for new instruments and methods that will enable and improve the quick and efficient identification of target particles. More particularly, there is a need for new instruments and methods that will enable and improve the ability to assess at an early stage and predict the efficacy of, for example, cancer treatment regimens, including traditional chemotherapy, molecularly targeted agents and immunotherapy, which can be obtained in accordance with the present inventions.

US 2012/0031759 A1 discloses a flow chamber according to the preamble of the independent claim 1.

WO 2013/028573 A discloses a gradient array dielectrophoresis separation (grads) with concomitant light therapy.

SUMMARY OF THE INVENTIONS

The invention is defined in the claims.

The present inventions are generally directed to instruments (or apparatuses), component devices or parts that can be employed in the instruments, and methods employing the instruments and/or their component parts, for processing samples of interest (e.g., separating target particles of interest from non-target particles). In one aspect, an apparatus generally includes an integrated fluidic device using dielectrophoresis in a continuous, semi-continuous, staged, or intermittent system, that includes a microfluidic chamber capable of enriching and isolating target particles, such as viable and/or fixed circulating tumor cells (CTCs) or disseminated tumor cells (DTCs), from a suspension of target and non-target particles (e.g., a suspension of cells). The apparatus enables the quantitative and/or qualitative analyzing of a subpopulation of the target particle-containing suspension (i.e., the target particles of the suspension), by using, for example, molecular analysis methods for diagnosis of tumoral conditions and/or corresponding state of advance of a disease state such as cancer.

In one aspect, the present disclosure provides a flow chamber having an interior surface and an exterior surface that define an area inside of the flow chamber for the direct or indirect flow of one or more fluids for the separation of a target particle. The flow chamber includes a floor and a ceiling and the area within the flow chamber is configured and positioned to receive a fluid, such as a fluidic suspension. The flow chamber includes one or more ports configured and positioned for introducing or receiving a first fluid, such as an elution buffer, from a position outside of the flow chamber through an exterior position of a wall surface of the flow chamber and into the flow chamber. The port(s) are coupled, directly or indirectly, to the flow chamber. A second port or series of ports, configured and positioned for introducing or receiving (e.g., infusing) a second fluid, such as a sample of interest to be processed and analyzed, from a position outside of the flow chamber through an exterior position of a wall surface of the flow chamber and into the flow chamber. The second port(s) are also coupled, directly or indirectly, to the flow chamber. Thus, upon infusion of the one or more fluid types, such as a sample of interest and an elution buffer, into the flow chamber a fluidic suspension is created.

In another aspect of the disclosure, the chamber may include two or more horizontally oriented, and/or vertically oriented, levels or stages that define two or more areas inside of the chamber. The stages may be separated by one or more channels of communication between each level. The two or more areas may be distinct, or remote, from each other, or they may be directly adjacent to allow for fluid communication between the internal chamber areas. The channels should be positioned and configured to allow for fluid communication or transfer of fluids between the two, or more, separation areas within a single chamber, or between one or more areas of one or more chambers within a single chamber, (e.g., two or more chambers), or separating various from one defined group of particles from the same defined group of particles that have subtle differences of interest.

In an aspect of the disclosure, a first fluid may be injected or infused into a first area of a separation and processing chamber. A second fluid may be injected or infused, although it is possible to release a suspension into a first chamber together. A first fluid infusion may be released directly into, below, above, or from the side, the first layer of the first chamber. Assuming two or more interior layers make up the chambers, the layers will be separated or combined as necessary to create or distinguish different batches, or redefined batches, of target particles. Thus, a series of two or more internal layers of one or more internal chamber areas may be positioned and configured to lead into a central or separate combining area(s), or out from one or more central or combining or separating area(s). The internal areas maybe be oriented and configured such that a first liquid, or a plurality of liquids, infused into the chamber (or the first layer in a series of chambers) will be subject to one or more electromagnetic forces that will assist or result in the removal of at least one set of target cells or particles from another set of target cells or particles, or from a remainder of a suspension.

Thus, a fluidic suspension (e.g., sample and buffer) may be created or combined and formed in a first portion of an area of a first chamber, or one or more chamber(s) may be employed to combine fluids infused from the same or different port(s) of one or more chambers, after which they may be separated. Alternatively, a chamber at a first level, and elements of a first level of the combination, such as a first portion of target particles of interest, may be transported from the first area (or level or stage) of a chamber into a second area (or level or stage) of the chamber. Thus, the interior of the (one or more) chamber(s) may each independently define two or more internal areas (or sub-chambers) for the processing of fluids. Target particles of interest may be separated from non-target particles, or from one or more groups of target particles through a cascade or series of interconnected flow chambers or process events designed to further distinguish, classify, or process the separated particles. These aspects allow for simultaneous separation and processing of similar or distinct groups of target particles.

The areas within the chambers for providing levels or separate areas for distinguishing particles may be in communication through slits that may be oriented perpendicularly to the direction of flow or at some other angle ranging from approximately one (1) degree above or below the other chamber area, through 179 degrees (e.g., from left to right or right to left). Thus, elbow passages or junctions of communication or transfer (e.g., conduits) between sets of electrodes that allow for transfer of particles between chamber channels or levels may be at angles of about 90 degrees, or the passages or junction areas may transfer fluids on slants or ramps (inclines or declines) of about 45 degrees, or about 30 degrees, or about 15 degrees, reflexive. Thus, the disclosure employs stages of separation that may be seen as a cascade wherein a first series of object and target cells flow through a first level while a second series of object cells are drawn into a second or tertiary level for separation. Electrodes may be positioned at floor levels of each chamber channel or alternatively, at ceiling levels of each chamber channel, or at floor levels in one chamber channel and at ceiling levels in another chamber channel, or in various combinations of floor and ceiling electrode arrangements (e.g., intermittently at the floor of an upper channel, and intermittently at the ceiling and/or floor of a lower channel). These new instruments and devices allow for separation of target particles that has not previously employed in order to obtain better separation of target particles, from secondary target particles, and tertiary target particles.

The one or more chambers include one or more electrodes, or series of electrodes although they may be employed in combination with the instrument or apparatus of the disclosure. The electrodes may be positioned next to (adjacent) or coupled to one or more floors (or ceilings) of the chamber(s). The electrode(s) of the claimed invention are affixed to a floor area of the flow chamber and they are configured and positioned to include a first zone that is associated with a first inter-electrode pitch that extends from a midpoint of one of a plurality of first conductive traces to a midpoint of an adjacent or associated first conductive trace in the first zone or inter-electrode gap, wherein the first zone is configured to generate a first electric field when a first electric signal is applied. A second zone is associated with a second inter-electrode pitch that extends from about a first area, such as a midpoint (or an initial area) of one of a plurality of second conductive traces, to a second area or midpoint of an adjacent or second conductive trace in the second zone or inter-electrode gap, wherein the second zone is configured to generate a second electric field with at least one of a different magnitude, and/or a different depth of field, and a different spatial profile than the first electric field when the electric signal is applied, and the first electric field and the second electric field generate dielectrophoretic forces that facilitate separating the target particle from the fluidic suspension via dielectrophoresis; a waste disposal port configured to discharge the fluidic suspension, where the waste disposal port may be coupled to the chamber; and a collection port configured to collect a target particle enriched sample, where the collection port may be coupled to the chamber.

Therefore, the disclosure provides aspects wherein there is one chamber having two levels and two flow areas in accordance with the disclosure. The two levels are in tandem, although they may be separated by one or more intervening levels. A first fluid containing a sample is introduced through a first port. A second fluid with no sample is introduced through a second port. The first and second fluids are connected via openings (ports) fabricated in the floor that divides the upper and lower fluid levels in the chamber. The flow rate of the second fluid is greater than the flow rate of first fluid. The first fluid introduced through the first port will be subject to a pressure difference that drives a portion of the first fluid and the sample into the second fluid in the lower level of the chamber through one or more port openings. Sample particles suspended in first fluid are introduced through the first port will be subject to a first electrode force as they travel along a path inside of the chamber. Particles in the first fluid that are subjected to a negative dielectrophoretic force will be repelled from the openings and prevented from entering the second fluid in the lower level. Particles in the first fluid that are subjected to a positive dielectrophoretic force will be attracted to the openings between levels and aided in their passage into the second fluid. Particles that have entered the second fluid through the openings are subject to a second electrode force at various points as they travels along a path inside of the chamber at the lower level. Particles in the second fluid that are subjected to a negative dielectrophoretic force will be repelled from the roof of the lower part of the chamber and directed down to the floor of the lower part of the chamber. The voltage frequency applied to give the electric force in the upper level to attract target particle(s) towards and through the openings by positive dielectrophoresis will differ from the voltage frequency applied to give the electric force in the lower level to push by negative dielectrophoresis the target particle(s) towards the floor of the lower chamber. Thus, depending upon the electric forces employed (strength and frequencies) particles traveling along the upper level will pass through an opening (one or more openings) and be attracted to the second lower level based upon their size and structural and/or dielectric features and/or properties that they may exhibit. As shown, target particles may be selectively separated through two or more openings in this example and directed towards the floor of the lower level of chamber.

Another aspect of the present disclosure, said aspect not being covered by the claims, provides an electrode adjacent to or affixed to or coupled to a floor of a flow chamber. The electrode includes a first zone that is associated with a first inter-electrode pitch that extends from the midpoint of one of a plurality of first conductive traces to the midpoint of an adjacent or first conductive trace in the first zone. The first zone is configured to generate a first electric field when an electric signal is applied; and a second zone that is associated with a second inter-electrode pitch that extends from the midpoint of one of a plurality of second conductive traces to the midpoint of an adjacent or second conductive trace in the second zone or second inter-electrode gap. The second zone is configured to generate a second electric field with or without at least one of a different magnitude, a different depth of field, and a different spatial profile than the first electric field when the electric signal is applied, wherein the first electric field and the second electric field generate dielectrophoretic forces.

In another aspect, the disclosure provides a method according to the independent claim 2.

The method includes

the steps of continuously introducing a sample containing target particles and non-target particles into the second port of the flow chamber;
continuously introducing an elution buffer into the first port of the flow chamber;
wherein the sample and the elution buffer form a fluidic suspension in a flow chamber; and exposing the fluidic suspension to an electric field in a defined region of the flow chamber.

The electric field is produced by an electrode coupled to the floor of the chamber, wherein the electrode comprises a first zone that is associated with a plurality of first conductive traces and a first inter-electrode gap that generate a first electric field when an electric signal is applied; and a second zone that is associated with a plurality of second conductive traces and a second inter-electrode gap that generate a second electric field of a different magnitude than the first electric field when the electric signal is applied, wherein the first electric field and the second electric field generate dielectrophoretic forces that separates the target particle from the fluidic suspension; and d) collecting the target particles.

This disclosure discloses a method to enhance the efficiency of separation of cancer cells from peripheral blood by means of dielectrophoresis in a continuous flow microfluidic device. The number of cancer cells in a typical blood sample is very low, typically less than 100 cancer cells per thousand million normal blood cells. Minimal dilution of the blood sample is required in order to reduce the time required for the cell separation process, and so the mean separation distance between cells in a blood sample is small. Because the cells are so closely packed together they can interact in ways that reduce the efficiency by which a dielectric force can selectively separate the target cancer cells from the normal blood cells. Steric hindrance is a particularly dominant cell-cell interaction that can reduce the efficiency of dielectrophoretic cell separation, and this deleterious influence can be enhanced by a special form of steric hindrance known as 'pearl chain' formation. Chains of cells can form as a result of dipole-dipole interactions between cells. On application of the electric field required to create the dielectrophoresis effect, the cells become electrically polarized and assume the character of small electrets - the electrical equivalent of micro-magnets. Polarized cells can attract each other to form chains of cells, and such chains are preferentially formed between cells having dielectric properties that are similar and therefore the most difficult to separate efficiently be means of dielectrophoresis. Most cells in a blood sample will have different dielectric properties to the cancer cells, and will be selectively repelled from the electrodes by negative dielectrophoresis. In an ideal situation the cancer cells will be selectively attracted to the electrodes by positive dielectrophoresis, but if they have been entrapped in pearl chains as a result of dipole-dipole interactions they can be repelled from the electrodes because the overall dielectrophoretic properties of the chain of cells differs from that of an isolated blood cell, and more critically also from that of an isolated cancer cell.

In this disclosure we describe how the dipole-dipole interactions between cells can be reduced whilst at the same time increasing the dielectrophoretic force that can selectively tease out the rare cancer cells from normal blood cells. This is achieved by careful selection of the width and spacing of the interdigitated conductive traces used for the electrodes, taking into account the typical size of the target cancer cell or other target particle to be selectively separated. In one aspect of this disclosure more than one set of the conductive traces are employed. If two sets are employed, the first set leads up to a fluid exit port from which target cells are extracted from the main input sample. Target cells that have escaped this first cell separation procedure are then carried by the fluid flow over the second set of conductive traces - which may have the same or a different width and spacing of conductive traces from the first set.

A further aspect of this disclosure is that the depth of the chamber floor on which the second set of conductive traces are fabricated may be greater than the chamber floor depth on which the first set of conductive traces are fabricated. The sample is injected into the chamber containing the first set of conductive traces at a level close to the chamber floor. The cells in the sample remain at this level unless they are repelled above the floor and into the main fluid flow stream by negative dielectrophoresis. However, cells close to the chamber floor can experience forces other than the dielectrophoretic force - and dominant examples of this include lift-off forces, cell adhesion forces and an electrostatic force of attraction as a cell passes over a conductive trace. All cells carry a net negative charge and when close to a conductive surface a 'mirror-image' charge of positive polarity is induced so as to electrostatically attract the cell to the conductive surface. By lowering the chamber floor on which the second set of conductive traces are fabricated there is a drop in fluid height after the exit port at the end of the first set of conductive traces. This drop in chamber floor depth can take the form of a step-like profile or a ramp-like profile, and be a depth of 10% to 20% of the total height between the floor and roof of the fluid chamber. This difference in chamber height can be achieved either by milling out the floor of the second chamber, or incorporating an extra layer of substrate onto the floor of the first chamber. Target cells that have escaped the first applied dielectrophoretic force are thus less likely to be influenced by fluid lift-off forces, and will be located in a weaker electric field where dipole-dipole forces and pearl chaining of cells will be reduced. An increased capture of target cells that have escaped separation over the first set of conductive traces can be achieved by an appropriate choice of the frequency and voltage signal applied to the second set of conductive traces, along with the spacing and width of these traces.

BRIEF DESCRIPTION OF THE DRAWINGS

The disclosure will be better understood, and features, aspects and advantages other than those set forth above will become apparent when consideration is given to the following detailed description thereof. Such detailed description makes reference to the following drawings.

FIG. 1 is an illustration of an exemplary electrode having different sections according to an embodiment of the present invention.

FIG. 2 is an illustration of one embodiment of a chamber in accordance with the invention.

FIG. 3 is an illustration of another embodiment of a chamber in accordance with the invention.

FIG. 4 is an illustration of another embodiment of a chamber in accordance with the invention.

FIG. 5 is an embodiment of one chamber having two levels and two flow areas in accordance with the invention.

FIG. 6 is another example of an electrode in accordance with the invention.

FIG. 7 is another example different design of a chamber for co-mingling particles or separating particles in accordance with the present invention.

FIG. 8 is a graph showing the recovery of SKOV3 ovarian cancer cells at various flow rates for electrode geometries E1 and E2 as discussed in Example 1.

FIG. 9 is a graph showing a comparison of the average peripheral blood mononuclear cell (PBMC) reduction for electrode geometries E1 and E2 as discussed in Example 1.

FIG. 10 illustrates a comparison of the results obtained by the electrode present invention with respect to different electrode geometries and the levitation height.

DETAILED DESCRIPTION OF THE DISCLOSURE

Unless defined otherwise, all technical and scientific terms used herein have the same meaning as commonly understood by one of ordinary skill in the art to which the disclosure belongs. Although any methods and materials similar to or equivalent to those described herein can be used in the practice or testing of the present disclosure, the preferred methods and materials are described below.

In accordance with the present disclosure, methods and apparatuses conccrning an integrated fluidic device using diclcctrophorcsis in a continuous flow microfluidic chamber capable of separating, enriching and/or isolating a target particle are disclosed. The methods and apparatus of the present disclosure advance existing dielectrophoretic methods and apparatus for the discrimination of particulate matter and solubilized matter of different types in a fluidic suspension enabling subsequent analysis of rare cells. In particular, the methods and apparatus of the present disclosure use dielectrophoresis in a continuous flow microfluidic chamber capable of enriching and isolating viable and/or fixed circulating tumor cells (CTCs) or disseminated tumor cells (DTCs) from a suspension of cells thereby enabling quantitative or qualitative analysis of that subpopulation using molecular analysis methods for diagnosis of tumoral conditions and/or corresponding state of advance of the cancer, whereby the sample is preferably obtained using minimally invasive methods. The advances relate to a combination of unique electrode patterns designed to more effectively affect desired movement of target particles flowing over electrode and specific operating conditions to further control target particle movement so as to yield target particles with sufficient recovery efficiency and purity. Particularly with respect to CTCs and DTCs, the combination of unique electrode patterns designed to more effectively affect desired movement of CTCs and DTCs flowing over electrode and specific operating conditions to further control CTCs and DTCs movement so as to yield target particles with sufficient recovery efficiency and purity to enable multiple methods of molecular analysis using CTCs and DTCs obtained from cancer patient blood samples whereas existing molecular analytical methods require tumoral cells obtained via more invasive tissue biopsies.

The methods of the present disclosure for the enrichment and isolation of target rare cells is both high throughput and cell-labeling independent. This approach of the present disclosure is based on dielectrophoresis (DEP) in a continuous flow microfluidic chamber for antibody independent isolation of rare CTCs and cancer stem cells from whole blood. By enriching for rare cells from blood, it allows for the analysis of disease state. The method described herein allows significant enrichment of the target cell populations so that these rare cells are amenable to analysis with current protein, DNA, RNA detection methods.

The cell isolation technology of the present disclosure is an improved approach from the state of the art, in that it introduces a novel electrode design which varies width and spacing of the electrical traces of an interdigitated electrode to create different zones which generate dielectric forces of differing intensity, magnitude, depth of field and/or spatial profile, thereby obviating the need for multiple signal generators of the particles flowing over the electrode. The design enables antibody free or magnetic bead-free isolation of live cancer cells from a wide range of human cancers, including non-epithelial cancers or cancers with low or negative epithelial cell adhesion molecule (EpCAM) expression. Since the isolated cells do not need to be modified (i.e., no labeling or fixing), the cells can be cultured and analyzed for RNA/DNA and protein analysis for complete cell characterization. The capture of viable, unlabeled CTCs from cancer patients will contribute to a significant improvement for diagnosis, prognosis and discovery of biomarkers associated with cancer progression and treatment, thereby facilitating personalized medicine.

Housing/Chamber and Electrodes

In one aspect, the present disclosure is directed to a flow chamber for separating at least one material from another material using dielectrophoresis. The flow chamber includes a chamber with a floor, walls to contain the fluids and a ceiling that allows an elution buffer to continuously enter through a flow port and travel through the flow chamber at a controlled rate. The flow chamber allows a sample, e.g., without limitation, a cell suspension, containing a plurality of target particles and a plurality of non-target particles to be introduced through an infusion port. The sample enters into the chamber through an infusion port at a controlled flow rate and mixes with the elution buffer to form a fluidic suspension. As the fluidic suspension travels through the flow chamber, the fluidic suspension experiences dielectric forces generated by a non-uniform electric field created by an interdigitated electrode positioned on the floor of the chamber and connected to an electrical signal generator. Dielectric conditions can be established to simultaneously exert a positive (attractive) force on the target particle, and to exert a negative (repulsive) force on non-target particles.

The flow chamber includes an interdigitated electrode coupled to the floor of the chamber. The electrode can be made of highly conductive materials known by those skilled in the art. Suitable conductive materials can be, for example, gold, copper or platinum, or titanium, or combinations. In one implementation, the electrode is made of gold, titanium, and/or platinum to reduce cell toxicity caused by the electrode. The electrode can be fabricated using processes such as, for example, lamination, evaporation, electroplating, laser ablation and chemical etching over which an applied electrical signal voltage is constant.

When the electrode is powered by an AC sinusoidal voltage, the electrode creates a spatially inhomogeneous stationary AC electric field, which causes dielectrophoresis induced movement on the suspended particles in the sample. This dielectric force will have a component acting in a direction normal to the electrode plane, causing target particles to move towards the electrode plane and non-target particles to move away from the electrode plane. When the dielectric force is used in combination with a fluidic suspension in the flow chamber, the dielectric force can be oriented in a direction normal to the fluidic suspension causing particles to occupy different heights in the channel. The height of the particles is dependent on the direction and magnitude of the force, which are a function of the electrode geometry, applied voltage and the relative polarizability of the suspended particles. In an example embodiment, the electrode can include at least two zones configured to generate dielectric forces of different intensities. In the example embodiment, the sample experiences a first dielectric force while flowing through the first zone of the chamber, and experiences a second dielectric force while flowing through the second zone of the chamber. More specifically, the DEP generated by the first zone has a shorter depth of field to limit dipole interactions with the target cells, whereas the second zone has a longer depth of field to achieve higher levitation of the non-target cells. By increasing the trace width and shortening the inter-electrode gap width, the target cells experience stronger DEP attraction towards the electrode plane enabling higher recovery at higher fluid flow rates.

By precisely controlling the elution buffer flow rate and sample infusion rate, and by introducing the sample into the chamber through a slit on the bottom of the chamber, the target particles stay close to the floor until molecules (including target particles) in the sample encounter an attractive or repulsive DEP force. To improve the effectiveness of DEP based separation, multiple zones are incorporated within a common electrode to create different force characteristics in different regions using a single signal generator. This approach eliminates the requirements for multiple signal generators and multiple separately connected electrode elements while providing multiple zones with different DEP force characteristics.

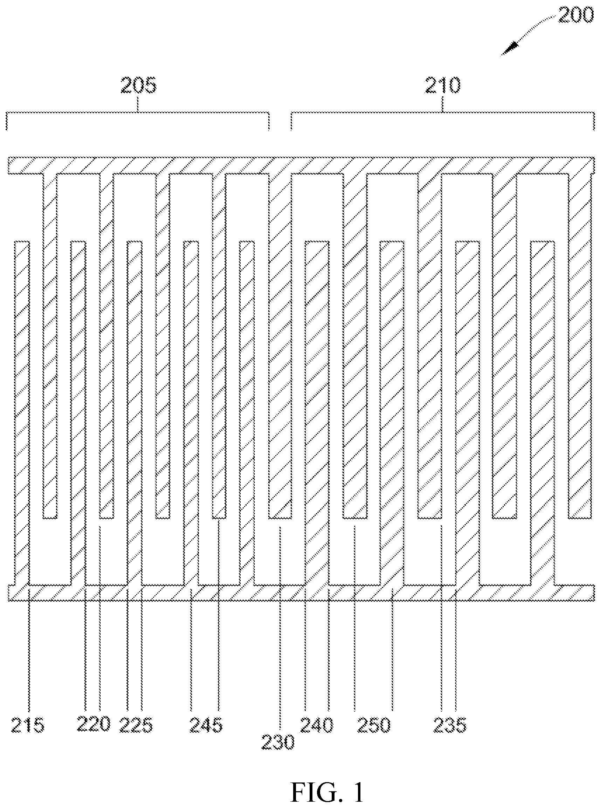

FIG. 1 illustrates one embodiment of an electrode 200 for use in generating dielectric forces according to an embodiment of the present disclosure. In the example embodiment, electrode 200 is comprised of at least two zones, a first zone 205 and a second zone 210. In the first zone 205, electrode 200 is defined by a plurality of first conductive traces 215 spaced apart from each other by a first inter-electrode gap 220. Each of the first conductive traces 215 in first zone 205 are of a first conductive trace width 225. In the second zone 210, electrode 200 is defined by a plurality of second conductive traces 230 spaced apart from each other by a second inter-electrode gap 235. Each of the second conductive traces 230 in second zone 210 is of a second conductive trace width 240. In the exemplary embodiment, first conductive traces 215 and first inter-electrode gap 220 define a first electrode or first inter-electrode pitch 245 and second conductive traces 230 and inter-electrode gap 235 define a second electrode or second inter-electrode pitch 250. 250 extends from the midpoint of an inter-electrode gap 225, 235 to the midpoint of an associated conductive trace 215, 230. In some embodiments, electrode 200 may utilize three or more zones, each zone being associated with a respective inter-electrode gap and plurality of conductive traces.

According to the invention, the first conductive trace width 225 and the second conductive trace width 240 are of different widths such that the dielectric forces exerted on a fluidic suspension are of a differing magnitude in the first zone 205 and the second zone 210 respectively. A single signal generator may apply a single voltage to both first zone 205 and second zone 210 and generate dielectric forces of differing intensity, magnitude, depth of field and/or spatial profile, thereby obviating the need for multiple signal generators. Further in the example embodiment, first inter-electrode gap 220 and second inter-electrode gap 235 have the same width such that the dielectric forces are controlled by the first conductive trace width 225 and second conductive trace width 240. In another implementation not covered by the claims, the first inter-electrode gap 220 and the second inter-electrode gap 235 are of different widths such that the dielectric forces exerted on a sample are of a differing magnitude in the first zone 205 and the second zone 210 respectively. In such an implementation, the single signal generator may apply a voltage to both first zone 205 and second zone 210 to generate dielectric forces of differing intensity, magnitude, depth of field and/or spatial profile, thereby obviating the need for multiple signal generators. Further in such an implementation first conductive trace width 225 and second conductive trace width 240 may be the same width or different widths.

For a fixed volume fraction of suspended cells of similar dielectric properties, then according to Kadaksham et al, in their publication 'Dielectrophoresis induced by clustering regimes of viable yeast cells', Electrophoresis, Vol. 26, pages 3738-3744, 2005, the ratio of the dipole interaction force FD between cells to the dielectrophoretic force FDEP acting on the cells depends on the ratio:

$$\frac{F_D}{F_{DEP}} \propto \frac{L}{a} = P$$

where L is the inter-electrode gap 220, 235 and a is the cell radius. If parameter P is of order 1, the dipole-dipole interactions between cells and the DEP forces are of comparable magnitude. In this case, we expect cells to form chains of cells. According to Sancho et al, in their publication 'Interaction between cells in dielectrophoresis and electrorotation experiments', Biomicrofuidics, Vol 4, 022802, 2010, the chaining of cells alters the electrical signal frequency at which the cells make the transition between being repelled or attracted towards an electrode. This reduces the efficiency at which target cells can be removed non-target cells. However, if parameter P is less than order 1, dipole-dipole interactions between cells are negligible and we expect cells to move individually and to exhibit their natural DEP collection characteristics. Hence, by reducing the value of parameter P the target particles can be individually manipulated with minimal interference from non-target particles. We find that for the volume fraction of suspended cells typically used in our cell separation protocols that the ratio of the dipole interaction force to the DEP force for electrode geometry with an inter-electrode gap 220, 235, of 30 microns is 1.6 times lower than for electrode geometry with an inter-electrode gap 220, 235, of 50 microns. This enables highly selective and individual manipulation of the target particles enabling higher recovery.

The inter-electrode gap 220, 235, of 30 microns according to the invention applies to cells of diameter around 10 microns. If particles such as viral capsids, for example, of sizes of the order 0.1 micron are to be separated by DEP according to the method of this invention, in order to keep the parameter P less than order 1 an inter-electrode gap 220, 235 of less than 0.5 micron and preferably around 0.3 microns should be adopted. Likewise for particles such as bacteria of size around 1 micron, an inter-electrode gap 220, 235 of 3 microns is preferable. For particles or cells larger than around 100 microns in size the inter-electrode gap 220, 235, should be greater than 30 microns.

Additionally, in the example embodiment, first zone 205 is upstream of second zone 210 and closer to the infusion port. Second zone 210 is downstream of the first zone and closer to the collection slot. As a result of forces diminishing rapidly with height and with smaller gaps and traces, the width of conductive traces in the second zone (240) or the width of at least one of a second inter-electrode gap (235) and second conductive traces (230) in the second zone (210) is varied with respect to the conductive trace widths in the first zone 215 or at least one of the first inter-electrode gap (220) and the first conductive traces in order to extend the reach of the repulsive forces, thereby pushing the non-target particles contained in the sample further away from electrode 200 into a waste disposal port.

According to the invention, first zone 205 has first inter-electrode gap 220 of 30 microns and first conductive trace width 215 of 30 microns, and second zone 210, has a second inter-electrode gap 235 of 30 microns and a second conductive trace width 240 of 50 microns.

By increasing the second conductive trace width 240 and retaining the width of second inter-electrode gap 235, the target particles experience stronger DEP attraction towards electrode 200 and the non-target particles experience stronger DEP repulsion away from electrode 200, enabling higher purities of target particles to be collected at higher fluid flow rates. In another example not covered by the claims, first zone 205 may have a first inter-electrode gap 220 of about 30 microns and first conductive trace width 225 of about 30 microns, and second zone 210 may have a second inter-electrode gap 235 of about 20 microns and a second conductive trace width 240 of about 30 microns. By decreasing the width of inter-electrode gap 235 and retaining second conductive trace width 240; the target particles experience stronger DEP attraction towards electrode 200 and non-target particles experience stronger DEP repulsion away from electrode 200. In such examples, the dielectrophoretic forces generated by first zone 205 have a shorter depth of field to limit dipole interactions with the target particles, whereas the second zone has a longer depth of field to achieve higher levitation of the non-target particles. In other implementations, inter-electrode gaps 220, 235 and conductive trace widths 225, 240 may be any dimension that enables electrode 200 to function as described here. By increasing the second conductive trace width 240 and retaining the width of second inter-electrode gap 235, the non-target particles experience stronger DEP repulsion away from electrode 200 enabling higher recovery at higher fluid flow rates and thus yielding higher levels of purity.

The inter-electrode gaps 220, 235 and conductive trace widths 215, 240 of electrode 200 affect the levitation height of cells undergoing dielectrophoresis. With narrower gaps, the dielectric forces are higher near the surface of electrode 200, but decay more rapidly as distance from the surface of electrode 200 increases, causing reduced levitation. Dipole interactions in concentrated particle suspensions are influenced by the inter-electrode gaps 220, 235 and conductive trace (225) or inter-electrode widths 235, 240. These interactions can compromise the selective performance of dielectrophoresis based separation. Therefore, in at least one embodiment, electrode 200 or the first zone of electrode 200 includes a minimum or reduced particle interaction zone 205 as the target particle comes under the influence of a polarizing electric field. The reduced particle interaction zone reduces dielectrophoretic forces on the target particle as the target particles come under the influence of a polarizing electric field.

The dielectric force scales depend on the applied voltage, electrode gap (220, 235), particle radius and the frequency-dependent dielectric properties. The DEP force acting on spherical dielectric particles is given by

$$F_{DEP} = 2\pi\varepsilon_m Re[CM]R^3\nabla|E_{rms}|^2$$

$$\nabla|E_{rms}|^2 \propto \frac{V^2}{d^3}$$

where R is the radius of the cell, $\varepsilon_m$ is the absolute permittivity of the medium, CM is the Clausius Mossotti factor, V is the applied voltage, d is the gap between the electrodes and $E_{rms}$ is the root mean square value of the electric field.

The inter-electrode gap 220, 235 is considered to be a dominant scaling factor. Hence, by reducing the inter-electrode gap 220, 235 from 50 microns to 30 microns, the DEP force can be increased by a factor of 4.6 resulting in significantly lower velocity of cells travelling immediately above the electrode plane. Considering a two dimensional model with stable forces, the steady state velocity in the horizontal (x) and vertical (y) directions are given by

$$v_x = \frac{F_{DEPx}}{6\pi\eta r} + u_x \ , \quad v_y = \frac{F_{DEPy}}{6\pi\eta r} - \frac{2r^2}{9\eta}(\rho_p - \rho_m)g$$

where $\eta$ is the viscosity of the medium, $\rho_p$ and $\rho_m$ are the densities of the particle and medium respectively and g is the acceleration of gravity. Higher DEP forces enable the use of higher volumetric flow rates in the main flow channel while maintaining good target particle recovery.

The electrode 200 can be fabricated on a flex circuit. The flex circuit can be laminated to the flow chamber base to form the floor of the flow chamber. In other embodiments, the electrode trace pattern can be applied directly to the flow chamber base. In such an embodiment, the transition of target particles from the infusion port to the collection port can be improved by eliminating a layer in the flow path and allow loosening of positional tolerances since the need to align the flex precisely to the chamber base would be eliminated.

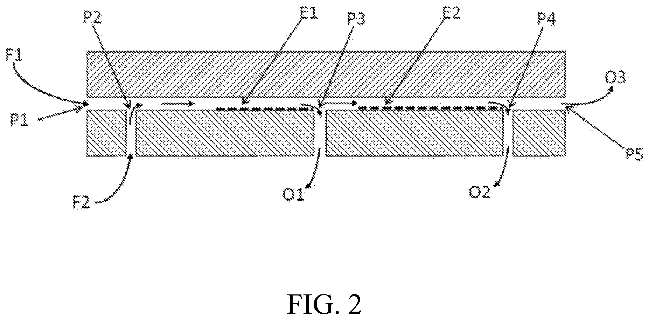

As illustrated in FIG. 2 the flow chamber has two sets of electrodes in series and two collection ports. A first fluid F1 with no sample (e.g., F1 is an elution buffer) is introduced through inlet port P1 at a continuous flow rate and establishes laminar flow conditions in the flow chamber. A second fluid F2 containing a sample is introduced through inlet port P2 which is located on the bottom of the flow chamber at a second continuous flow rate. As fluid F2 enters the flow chamber, the sample particles contained in F2 become entrained in the flow stream of F1 and are carried through the chamber.

By maintaining a balance of flow rates of F1 and F2, the particles remain near the floor of the flow chamber. In the initial region of the flow chamber ions diffuse from high conductivity regions to low conductivity regions to establish an equilibrium state of conductivity prior to reaching the first electrode E1. The first electrode E1 is connected to a signal generator which outputs a sinusoidal signal to create a non-uniform electric field which in turn affects a dielectrophoretic response in the particles. At specific frequencies of the signal, the target particles are positively attracted to the electrode while non-target particles are simultaneously repelled. The target particles are drawn through collection port P3 which is connected to a pump. The pump connected to collection port P3 operates at a constant flow rate to skim the bottom layer of fluid containing the target particles O1 .

The remaining fluid in the flow chamber continues downstream where it encounters a second electrode E2. The second electrode E2 is energized in a likewise fashion as the first electrode E1. It induces a dielectrophoretic response in the remaining particles and enables collection of any remaining target particles through collection port P4 which is also connected to a pump which operates at a constant flow rate to skim the bottom layer of fluid containing remaining target particles O2. The remaining fluid continues to flow downstream of collection port P4 and exists through a waste port P5.

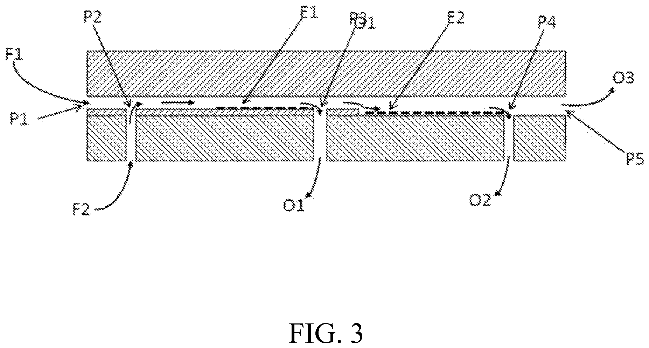

As illustrated in FIG 3, a flow chamber has two sets of electrodes in series and two collection ports. A first fluid F1 with no sample (e.g., F1 is an elution buffer) is introduced through inlet port P1 at a continuous flow rate and establishes laminar flow conditions in the flow chamber. A second fluid F2 containing a sample is introduced through inlet port P2 which is located on the bottom of the flow chamber at a second continuous flow rate. As fluid F2 enters the flow chamber, the sample particles contained in F2 become entrained in the flow stream of F1 and are carried through the chamber.

By maintaining a balance of flow rates of F1 and F2, the particles remain near the floor of the flow chamber. In the initial region of the flow chamber ions diffuse from high conductivity regions to low conductivity regions to establish an equilibrium state of conductivity prior to reaching the first electrode E1. The first electrode E1 is connected to a signal generator which outputs a sinusoidal signal to create a non-uniform electric field which in turn affects a dielectrophoretic response in the particles. At specific frequencies of the signal, the target particles are positively attracted to the electrode while non-target particles are simultaneously repelled. The target particles are drawn through collection port P3 which is connected to a pump. The pump connected to collection port P3 operates at a constant flow rate to skim the bottom layer of fluid containing the target particles O1 .

The remaining fluid in the flow chamber continues downstream where the flow chamber undergoes a stepped transition in the floor of the chamber resulting in a reduction in flow velocity. After the stepped transition, the fluids encounter a second electrode E2. The second electrode E2 is energized in a likewise fashion as the first electrode E1. It induces a dielectrophoretic response in the remaining particles and enables collection of any remaining target particles through collection port P4 which is also connected to a pump which operates at a constant flow rate to skim the bottom layer of fluid containing remaining target particles O2. The remaining fluid continues to flow downstream of collection port P4 and exists through a waste port P5.

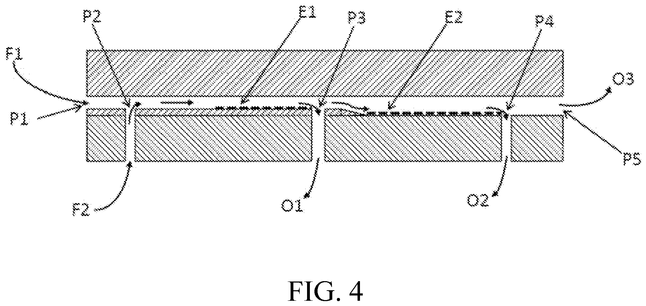

As illustrated in FIG 4, another embodiment of a flow chamber has two sets of electrodes in series and two collection ports. A first fluid F1 with no sample (elution buffer) is introduced through inlet port P1 at a continuous flow rate and establishes laminar flow conditions in the flow chamber. A second fluid F2 containing a sample is introduced through inlet port P2 which is located on the bottom of the flow chamber at a second continuous flow rate. As fluid F2 enters the flow chamber, the sample particles contained in F2 become entrained in the flow stream of F1 and are carried through the chamber.

By maintaining a balance of flow rates of F1 and F2, the particles remain near the floor of the flow chamber. In the initial region of the flow chamber ions diffuse from high conductivity regions to low conductivity regions to establish an equilibrium state of conductivity prior to reaching the first electrode E1. The first electrode E1 is connected to a signal generator which outputs a sinusoidal signal to create a non-uniform electric field which in turn affects a dielectrophoretic response in the particles. At specific frequencies of the signal, the target particles are positively attracted to the electrode while non-target particles are simultaneously repelled. The target particles are drawn through collection port P3 which is connected to a pump. The pump connected to collection port P3 operates at a constant flow rate to skim the bottom layer of fluid containing the target particles O1 .The remaining fluid in the flow chamber continues downstream where the flow chamber undergoes a sloped transition in the floor of the chamber resulting in a reduction in flow velocity.

After the sloped transition, the fluids encounter a second electrode E2. The second electrode E2 is energized in a likewise fashion as the first electrode E1. It induces a dielectrophoretic response in the remaining particles and enables collection of any remaining target particles through collection port P4 which is also connected to a pump which operates at a constant flow rate to skim the bottom layer of fluid containing remaining target particles O2. The remaining fluid continues to flow downstream of collection port P4 and exists through a waste port P5.

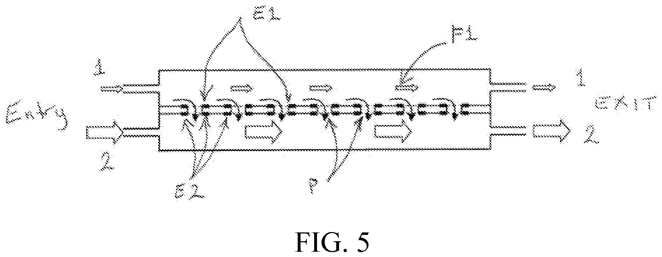

As illustrated in FIG 5, another embodiment of one chamber having two levels and two flow areas in accordance with the invention is presented. The two levels are shown in tandem, although they may be separated by one or more intervening levels. A first fluid F1 containing a sample is introduced through entry area (1). A second fluid F2 with no sample is introduced through entry area (2). Fluids F1 and F2 are connected via openings (ports) P fabricated in the floor that divides the upper and lower fluid levels in the chamber. The flow rate of fluid F2 is greater than the flow rate of fluid F1.

Fluid F1 introduced through area (1) will be subject to a pressure difference that drives fluid F1 and the sample into fluid F2 in the lower level of the chamber through openings P. Sample particles suspended in fluid F1 introduced through area (1) will be subject to a first electrode force (E1) as they travel along a path inside of the chamber. Particles in fluid F1 that are subjected to a negative dielectrophoretic force will be repelled from the openings and prevented from entering fluid F2 in the lower level. Particles in fluid F1 that are subjected to a positive dielectrophoretic force will be attracted to the openings P and aided in their passage into fluid F2. Particles that have entered fluid F2 through the openings P are subject to a second electrode force (E2) at various points as they travels along a path inside of the chamber at the lower level. Particles in fluid F2 that are subjected to a negative dielectrophoretic force will be repelled from the roof of the lower part of the chamber and directed down to the floor of the lower part of the chamber. The voltage frequency applied to give the electric force E1 in the upper level to attract target particle(s) towards and through the openings P by positive dielectrophoresis will differ from the voltage frequency applied to give the electric force E2 in the lower level to push by negative dielectrophoresis the target particle(s) towards the floor of the lower chamber. Thus, as shown, depending upon the electric forces employed (strength and frequencies) particles traveling along the upper level will pass through an opening (one or more openings) and be attracted to the second lower level based upon their size and structural and/or dielectric features and/or properties that they may exhibit. As shown, target particles may be selectively separated through two or more openings in this example and directed towards the floor of the lower level of chamber.

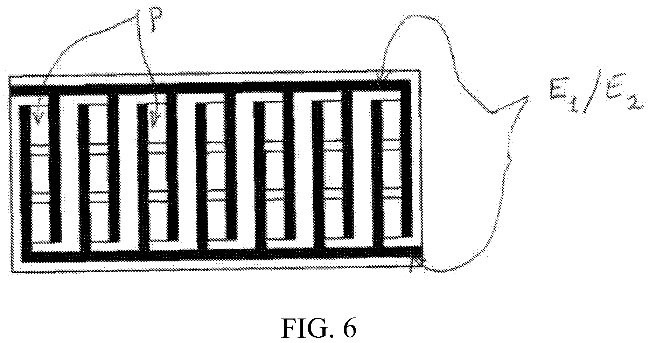

As illustrated in FIG. 6, another embodiment of an electrode design of the invention has two electrodes E1 and E2 fabricated at the edges of the openings P. The openings shown here take the form of rectangular slits, but they may be of any shape, such as circular in shape. The electrodes in this example are shown as interdigitated electrodes, but they may take the form of circular elements surrounding circular openings.

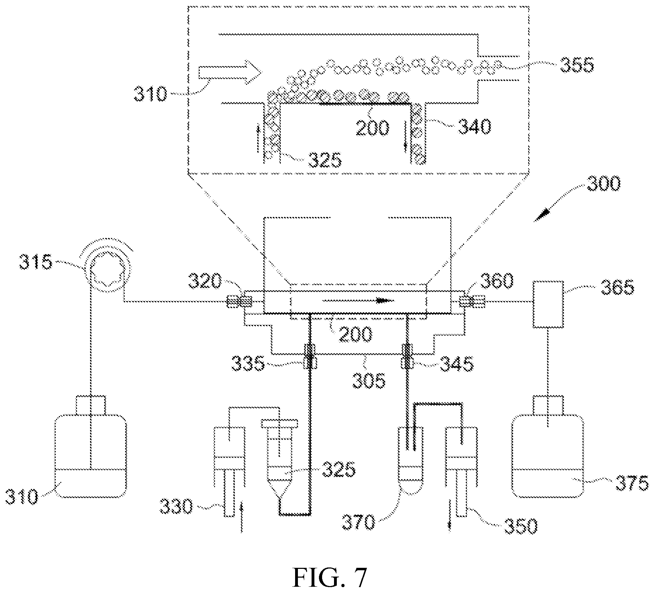

As illustrated in FIG. 7, a flow chamber system 300 is used in isolating a material according to an embodiment of the present disclosure. Flow chamber system 300 includes electrode 200, a flow chamber 305, an elution buffer 310, a pump 315, an elution buffer port 320, a sample 325, an injector 330, an infusion port 335, a target particle 340, a collection port 345, an aspirator 350, a non-target particle 355, a waste disposal port 360, a particle counter 365, a target particle enriched sample 370, and a target particle depleted sample 375.

In operation, flow chamber 305 is configured to receive a flow of an elution buffer 310 through elution buffer port 320. The elution buffer may have its rate of flow controlled by pump 315. Flow chamber 305 is further configured to receive sample 325 through infusion port 335. In the example embodiment, sample 325 includes target particles 340 and non-target particles 355. The flow rate of sample 325 through infusion port 335 is controlled by injector 330. Elution buffer 310 and sample 325 mix to form a fluidic suspension that travels downstream through flow chamber 305. The fluidic suspension experiences an electric field generated by electrode 200 (e.g., dielectrophoretic), the electric field having varying intensities in a first zone 205 (shown in FIG. 1) and a second zone 210 (shown in FIG. 1) of electrode 200. The varying electric field intensities generate varying intensities of dielectric forces on the fluidic suspension.

In one embodiment, the dielectric forces applied to sample 325 facilitate separating sample 325 into target particles 340 and non-target particles 355 Target particles 340 experience attractive dielectric forces and move toward electrode 200 positioned on the floor of flow chamber 305 and through collection port 345. An aspirator 350 may control the flow of target particles 340 through collection port 345. The material collected through collection port is a target particle enriched sample 370. Non-target particles 355 experience negative dielectric forces repelling non-target particles 355 away from electrode 200 and out through a waste disposal port 360. The material collected through waste disposal port 360 is as a target particle depleted sample 375. The dielectric forces facilitate separating target particle 340 and non-target particles 355 such that target particle enriched sample 370 has a higher ratio of target particles 340 to non-target particles 355 than target particle depleted sample 375.

In some embodiments, a particle counter 365 may be coupled to at least one of waste disposal port 360 and collection port 345. Particle counter 365 may be configured to count the particles exiting flow chamber 305, and may facilitate determining a percentage of target particles 340 recovered, and a ratio of target particles 340 to non-target particles 355.

In one embodiment, flow chamber 305 is oriented in a horizontal direction such that elution buffer 310 and sample 325 travel through flow chamber 305 in a horizontal direction. In an alternative embodiment, flow chamber 305 is oriented in a vertical or sloped direction such that elution buffer 310 and sample 325 travel through flow chamber 305 in a vertical or sloped direction (as opposed to horizontal direction) thereby eliminating or reducing any sedimentation force component.

In one embodiment, the height of flow chamber 305 relative to the width and length of flow chamber 305 is very small. Therefore, flow chamber 305 may establish laminar flow with a parabolic flow profile such that the flow velocity of elution buffer 310 and sample 325 is slower at the floor and ceiling and higher halfway between the floor and ceiling.

Also, in the example embodiment, the flow rate of elution buffer 310 and sample 325 is controlled to ensure target particles 340 introduced into flow chamber 305 stay near electrode 200 as they move through flow chamber 305.

Further, in the example embodiment, the floor of flow chamber 305 that is upstream of electrode 200 and closer to infusion port 335 may include at least one surface feature. In the example embodiment, the at least one surface feature includes a bump, post, ridge, and/or dimple that facilitates mixing sample 325 and elution buffer 310. The at least one surface feature may be formed by laser etching, embossing, injection molding, laser ablation, and/or any other method that enables the surface feature to function as described herein. In at least some implementations, a plurality of surface features are defined in the floor of flow chamber 305 to form a chevron pattern. In other implementations, the surface features may define a sine wave pattern, a serpentine pattern, and/or any other pattern that enables the surface features to facilitate mixing sample 325 and elution buffer 310. Alternatively, the floor of flow chamber 305 may be smooth upstream of electrode 200.

Methods of Using the Flow Chamber and Electrodes

In another aspect, the present disclosure is directed to a method for separating a target particle in a fluidic sample using dielectrophoresis in the flow chamber described above. The method includes: a) continuously introducing a sample 325 containing the target particle into a fluidic infusion port 335; b) continuously introducing an elution buffer 310 into a flow port 320; wherein the sample 325 and the elution buffer 310 form a fluidic sample in a flow chamber 305; c) applying an electric field to the fluidic sample in a defined region of the flow chamber, wherein the electric field is produced by an electrode 200 coupled to a floor of the flow chamber 305, wherein the electrode 200 comprises at least a first zone 205 and a second zone 210, wherein the first zone 205 is associated with a plurality of first conductive traces 215 and a first inter-electrode gap 220 and wherein the second zone 210 is associated with a plurality of second conductive traces 230 and a second inter-electrode gap 235, each zone of the electrode 200 configured to generate an electric field that separates the target particle from the fluidic sample by dielectrophoresis; and d) collecting the target particle 340.

As used herein, the terms "separating," "enriching," "isolating," and "purifying" are used interchangeably to refer to dividing the fluidic sample into constituent parts such that target particles are substantially free from non-target particles contained in the sample.

In another aspect, the present disclosure is directed to a method for separating a cell in a sample using dielectrophoresis in the flow chamber described above. The method includes: a) continuously introducing a sample 325 containing the cell into a fluidic infusion port 335; b) continuously introducing an elution buffer 310 into a flow port 320; wherein the sample 325 and the elution buffer 310 form a fluidic suspension in a flow chamber 305; c) exposing the fluidic suspension to an electric field in a defined region of the flow chamber, wherein the electric field is produced by an electrode 200 coupled to the floor of the chamber 305, wherein the electrode comprises: an electrode 200 coupled to a floor of the flow chamber 305, wherein the electrode 200 comprises at least a first zone 205 that is associated with a plurality of first conductive traces 215 and a first inter-electrode gap 220 that generate a first electric field when an electric signal is applied; and a second zone 210 that is associated with a plurality of second conductive traces 230 and a second inter-electrode gap 235 that generate a second electric field of a different magnitude than the first electric field when the electric signal is applied, wherein the first electric field and the second electric field generate dielectrophoretic forces that separates the target particle from the fluidic suspension; and d) collecting the target particle 340.

The target particle 340 is collected through a collection port 345 located at the bottom of the downstream end of the chamber 305, while the non-target particle 355 flows through a waste disposal port 360 located at the downstream end of the chamber 305. In particular, the target particle 340 to be isolated can be cancerous cells and the non-target particle 355 can be normal cells. However other materials, both organic and inorganic may be separated using the disclosed systems and methods. Furthermore, although described as a non-target particle 355 and a target particle 340, in some embodiments the non-target particle 355 collected through the waste disposal port 360 may include a desirable particle in addition to the target particles 340.

The elution buffer used in the methods described herein can have a conductivity of from about 5 mS/m to about 250 mS/m, including from about 5 mS/m to about 50 mS/m. A particularly suitable conductivity can be about 30 mS/m. The electric field can include an AC voltage of from about 2 Vp-p to about 8 Vp-p. The frequency of applied voltage can include from about 10 kHz to about 30 MHz. The elution buffer flow rate in the flow chamber is from about 0.5 mL/minute to about 5 mL/minute. The sample infusion rate in the flow chamber is from about 0.01 mL/minute to about 1 mL/minute.

The sample collection flow rate in the flow chamber can be from about 0.01 mL/minute to about 1 mL/minute.

The methods of the present disclosure will now be more particularly described. Initially, specimens can be taken from the peripheral circulation of a patient, or from bone marrow through various techniques known by those skilled in the art.

The proportion of tumor cells in the specimen taken can be enriched using various methods, such as for example: centrifugation on density gradient, constituted by solutions such as Ficoll or Percoll; mechanical enrichment, such as filters of various types; enrichment by means of separation by dielectrophoresis via a purposely provided device, for example, a dielectrophoretic-activated cell sorter (DACS); selective lysis, such as for example, selective lysis of erythrocytes not of interest; immunomagnetic separation, via immuno-magnetic beads with positive selection (using beads bound to specific antibodies for the population to be recovered) or with negative selection (depletion of cell populations that are not of interest), and in which the two types of selection can be coupled in order to increase the specificity of the procedure; FACS, on cells labeled with a specific fluorescent antibody; solid-phase immune-separation, advantageously by means of microfluidic systems presenting surfaces coated with specific antibodies for epithelial receptors (such as EpCAMs). For the majority, these procedures can be automated and all the sorting procedures can be preceded by separation by centrifugation on density gradient or alternatively they can be applied on whole blood.

Other techniques known to persons skilled in the art are MACS developed by Miltenyi Biotech and Easy-sep, developed by Stem-cell technologies. Alternatively, it is possible to use paramagnetic beads of larger dimensions that do not require the use of a special column, but can also be used when working with wells or test tubes (such as, for example, anti-EpCAM Dynabeads).

Enriching the specimen in the population of cells having at least one type of tumor cells can optionally be performed by selection of cells made on the basis of at least one parameter such as, for example, mass density; morphology; electrical properties; chemical properties; mechanical properties; expression of surface antigens; expression of intracytosolic antigens; dielectric properties; magnetic properties; geometrical properties (e.g., size); optical properties; and combinations thereof.

Next, the pre-enriched specimen containing the tumor cells is inserted in the microfluidic device including the flow chamber described above that is able to isolate them from other cell populations.

The methods according to the present disclosure can be used to discriminate target particles, including biological matter such as, for example, cells, cell organelles, cell aggregates, cell clumps, nucleic acids, proteins, amino acids, liquid globules, bacterium, protozoans, and viruses. Further, the biological matter can be, for example, a mixture of cell types, such as fetal nucleated red blood cells in a mixture of maternal blood. The biological matter can be, for example, cancer cells such as, for example, lung cancer, prostate cancer, melanoma, breast cancer, pancreatic cancer, liver cancer, neuroblastoma cancer, colorectal cancer, desmoid cancer, leukaemia, lymphoma, thyroid cancer, medulloblastoma, head and neck cancer, mesothelioma, uveal melanoma, bladder cancer, renal cancer, fibrofolliculomas, trichodiscomas, gastrointestinal polyps, skin squamous cell, skin basal cell, alveolar soft part sarcoma, osteosarcoma, ovarian, rhabdomyosarcoma and glioblastoma. The biological matter can be, for example, in a mixture with normal cells, or red blood cells infested with blood parasites such as malarial parasites.

Cells that are recovered using the apparatus and methods of the present disclosure can further be subjected to various analyses known to those skilled in the art. For example, cells can be subjected to protein, genetic, or chromosomal characterization, cell labeling such as, for example, anti-cytokeratin antibodies to identify cancer cells and labeling with CD45 to identify normal blood cells, fluorescence in situ hybridization probes (FISH), measuring their expression of markers of epithelial to mesenchymal transition, stem cell properties, PCR-based techniques, gene expression analysis, and combinations thereof.

The apparatus and methods of the present disclosure can also be used to guide drug dose selection by obtaining blood specimen from patient before and after treatment with a drug, enriching rare cells using the apparatus and methods described herein and measuring expression of pharmacodynamic markers in rare cells. The apparatus and methods of the present disclosure can also be used to obtain indications of a degree of drug response by enriching rare cells from blood, implanting rare cells into an animal, developing a xenograft tumor and studying its biology and/or drug response. The apparatus and methods of the present disclosure can also be used to obtain indications of a degree of drug response by enriching rare cells from blood and exposing them to various drugs ex vivo. The apparatus and methods of the present disclosure can also be used to evaluate the presence of deletions on CTCs obtained from a patient with prostate cancer by single-cell whole-genome amplification. The apparatus and methods of the present disclosure can also be used to identify the original tissue via the genetic profile and/or the profile of expression of the CTCs obtained from a patient with a cancer of unknown primary origin (CUPs), thus obtaining information that is fundamental for the choice of the most appropriate therapy. The apparatus and methods of the present disclosure can also be used to perform cell motility assays, invasion assays, and clonogenicity assays. The apparatus and methods of the present disclosure can also be used to perform analysis of gene expression on single CTCs and/or DTCs in order to identify prognostic indicators and potential therapeutic targets. The apparatus and methods of the present disclosure can also be used to isolate cells from blood, for example, prior to and/or after treatment with anti-cancer drugs such as chemotherapeutic or targeted therapeutic compounds, small molecules, monoclonal antibodies, immunotherapy, vaccines, gene therapy, nanoparticles, ribozyme-based therapeutics, viruses, radiation, surgery, thermal therapy, hormones, alkylating agents, antimetabolite, antibiotics, alkaloids/plant extracts, and combinations thereof.

The method can further include binding a cell and/or cells contained in the sample with a particle. Suitable particles can be, for example, a bead, an antibody, a nanoparticle, a colloid, a liposome, a metal and combinations thereof. In a particularly suitable embodiment, cells can be labeled with a magnetic particle coupled to antibodies having the following modalities: CD45 negative; CD45 and GPA negative; CD45 and CD14 negative; CD45, CD14 and GPA negative; EpCAM positive; CK positive; EGFR positive; Plectin positive; alpha feto protein positive; MUC1-positive; HER2-positive; and combinations thereof. In addition, the present invention will enable rapid identification of biomolecules, from which nucleic acids can be extracted, such as microbes, cells and virus.

The method further includes binding a cell and/or cells contained in the sample with a tracer. Suitable tracers can be, for example, a FISH probe, an antibody coupled to a flurophor, an antibody coupled to a chromogen, an antibody coupled to a microbead and combinations thereof. The antibody can be directed to an epitope that recognizes a protein such as, for example, EpCAM, cytokeratin 8, cytokeratin 18, cytokeratin 19, cytokeratin 20, CD45, EGFR, IGFR, PSA, TTF1, HER2/neu, MUC-1, Plectin, and combinations thereof.

Preferably, tumour cells are isolated to constitute a specimen to be analysed having a purity higher than 95%. Even more preferably, tumour cells are isolated to constitute a specimen to be analysed having a purity of 100%.

The tumour cells recovered according to the invention may be of various types, and various types of analyses can be performed on the cells that enable genetic or chromosomal characterization thereof at different levels of resolution and sensitivity and according to the diagnostic purpose of the study, in accordance with what has been described previously.

For example, it is possible to proceed to sequencing of CTCs taken from patients presenting metastases in order to detect the presence of mutations of the K-RAS gene. In the case of prostate cancer, it is possible to evaluate the presence of deletions on CTCs by means of single-cell whole-genome amplification. Again, in the case of cancers of unknown primary origin (CUPs) it is possible to identify the original tissue via the genetic profile and/or the profile of expression of the CTCs, thus obtaining information that is fundamental for the choice of the most appropriate therapy.

In the case of disseminated tumour cells, then, it is possible to perform analysis of gene expression on single CTCs and/or DTCs in order to identify prognostic indicators and potential therapeutic targets.

The methods of the present invention can also be used to discriminate particulate matter such as, for example, inorganic matter, such as minerals, crystals, liquid globules, colloidal, dielectric, conductive, semiconductive or insulating particles, and gas bubbles. Additionally, the methods of the present disclosure can be used to discriminate solubilized matter such as a molecule, or molecular aggregate, for example, proteins, or nucleic acids.

Particles to be discriminated may be any size. More particularly, particles of from about ~10 nm to about ~1 mm, and may include, for example, chemical or biological molecules (including proteins, DNA and RNA), assemblages of molecules, viruses, plasmids, bacteria, cells or cell aggregates, protozoans, embryos or other small organisms, as well as non-biological molecules, assemblages thereof, minerals, crystals, liquid globules, colloidal, dielectric, conductive, semi-conductive or insulating particles, and gas bubbles. For biological applications using living cells, the present disclosure allows cells to be separated without the need to alter them with ligands, stains, antibodies or other methods. Cells remain undamaged, unaltered and viable during and following separation. Non-biological applications similarly require no such alteration. It is recognized however, that the apparatus and methods according to the present disclosure are equally suitable for separating such biological matter even if they have been so altered.

The cells or target particles can be analyzed according to methods known by those skilled in the art. Suitable analysis can be, for example, sequencing, microsatellite analysis, comparative genomic hybridization (CGH), array CGH, end-point PCR, real-time PCR, methylation analysis, quantitative methylation analysis with pyrosequencing, bisulphite-genomic-sequencing PCR (BSP), methylation-specific PCR (MSP), combined bisulphite restriction analysis of DNA (COBRA), methylation-sensitive single nucleotide primer extension (MS-SNuPE), gene expression analysis, RT-PCR, single-cell gene expression, digital PCR, digital droplet PCR, ICE-cold PCR, PCR using labeled nucleotide primers such as SYBR GREEN®, PCR using molecular beacon probes such as TaqMan®, competitive allele-specific TaqMan® PCR (CAST-PCR), SNP TaqMan® PCR, and combinations thereof.

EXAMPLES

EXAMPLE 1

In this Example, the recovery of SKOV3 ovarian cancer cells was compared between electrode 200 (illustrated in FIG. 2) and electrode 100 (illustrated in FIG. 1) using different elution buffer flow rates.

Specifically, PBMC were spiked with SKOV3 ovarian cancer cells. The data collected utilized a prior art electrode with an inter-electrode gap 110 of 50 microns and a conductive trace width 115 of 50 microns. In this particular Example, electrode 200 had two zones 205, 210 with a first inter-electrode gap 220 of 30 microns, a first conductive trace width 225 of 30 microns, a second inter-electrode gap 235 of 30 microns and a second conductive trace width 240 of 50 microns.

Table 1 summarizes a comparison between the recovery of SKOV3 cancer cells using electrode 200 compared with electrode 100 using different elution buffer flow rates.

Table 1: Comparison of percent recovery with electrode configurations.

| Elution Buffer Flow Rate (mL/min) | % Recovery of SKOV3 ovarian cancer cells from the cellular sample using Electrode 100 | % Recovery of SKOV3 ovarian cancer cells from the cellular sample using Electrode 200 |
| --- | --- | --- |
| 1.5 | $64.8 \pm 3.5$ | $70.6 \pm 17.5$ |
| 1.8 | 68.6 | $75.4 \pm 5.7$ |
| 2.1 | $52.5 \pm 4.7$ | $81.3 \pm 7.8$ |
| 2.4 | $54.2 \pm 2$ | $68.6 \pm .2$ |
| 2.7 | $47.2 \pm 4.7$ | $70 \pm 8.6$ |

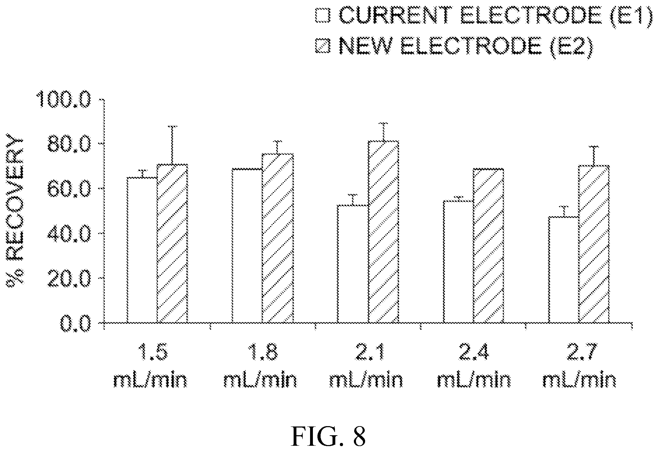

As summarized in Table 1 and illustrated in the graph shown in FIG. 8, electrode 200 exhibited improved recovery percentages of isolated SKOV3 ovarian cancer cells when compared with the prior art electrode 100.

Table 2 summarizes that the purity of isolated SKOV3 ovarian cancer cells obtained by comparing the ratio of SKOV3 ovarian cancer cells to waste material (PBMC) collected through collection port 345 was not adversely affected by employing electrode 200 of the present disclosure as compared to electrode 100.

Table 2: Ratio of Isolated Material to Waste Material.

| Elution Buffer Material Rate (mL/min) | Ratio of SKOV3 ovarian cancer cells to waste material collected through collection port using Electrode 100 | Ratio of SKOV3 ovarian cancer cells to waste material collected through collection port using Electrode 200. |
| --- | --- | --- |
| 1.5 | $521 \pm 320$ | $625 \pm 340$ |
| 1.8 | 906 | $2270 \pm 802$ |
| 2.1 | $5114 \pm 2411$ | $2508 \pm 121$ |
| 2.4 | $4462 \pm 2212$ | $6086 \pm 3970$ |
| 2.7 | $6833 \pm 4680$ | $2546 \pm 863$ |

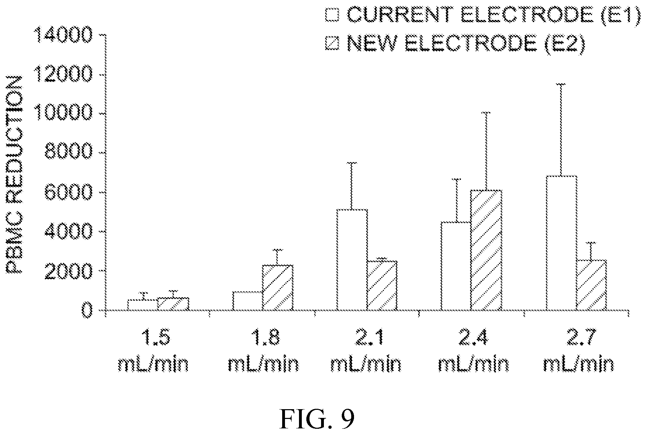

Experimentally, the cell velocity on electrode 100 was observed to be higher than electrode 200 resulting in cells escaping suction at the collection slot. Consequently, cell recovery on electrode 200 was higher than electrode 100 as shown in FIG. 9. The observed improvement in recovery could also be a result of lower dipole interaction as a result of the smaller gap in electrode pattern electrode 200.

Dipolar chaining of cells results in a cross-over frequency shift towards higher values. This can result in a decrease in recovery as a result of the target cells undergoing negative dielectrophoresis due to proximity interactions with non-target cells.

The data in FIG. 9 also shows that reduction in the electrode gap did not adversely affect the purity of the target cells (SKOV3 ovarian cancer cells) from a mixture (SKOV3 ovarian cancer cells and PBMCs). By employing an electrode trace of 50 microns and a gap of 30 microns, the levitation height is kept the same as that of an electrode with a trace and space of 50 microns. This ensured a high level of purity. The average PBMC reduction using electrode 100 and electrode 200 was greater than 2000 fold as shown in FIG. 9 and summarized in Table 2.

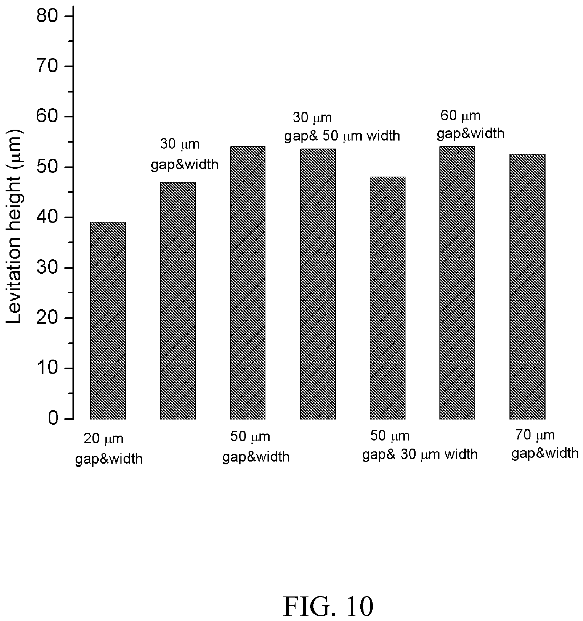

As illustrated in Figure 10, supporting data for the electrode design of the present invention compares a second interdigitated electrode zone containing traces with a 50 $\mu$m gap and 30 $\mu$m width. The data was generated from finite element simulations carried out in COMSOL Multiphysics. This method uses the equilibrium position generated from the force balance between the sedimentation and DEP forces to determine the levitation height. The voltage was kept constant at 4.5 Vpk-pk for the various electrode dimensions. The electrode gap and width were increased starting from a 20 $\mu$m gap and width up to a 70 $\mu$m gap and width. With regard to Figure 1, an interdigitated electrode zone with an inter-electrode width 240 and an inter-electrode gap 235 provides a similar levitation height as an interdigitated electrode geometry containing an inter-electrode gap 110 and a conductive trace width 115, thereby enabling the non-target particles to be levitated to a height of ~54 $\mu$m while reducing the effective length of the electrode structure by 20%.

The apparatus and methods of the present disclosure advance existing dielectrophoretic methods and apparatus for the discrimination of particulate matter and solubilized matter of different types in a fluidic suspension combined with subsequent analysis of rare cells. The advances relate to a combination of unique electrode patterns designed to more effectively affect desired movement of target particles flowing over the electrode and specific operating conditions to further control target particle movement so as to yield target particle with sufficient recovery efficiency and purity to enable multiple methods of molecular and other analysis. In particular, the apparatus and methods of the present disclosure use dielectrophoresis in a continuous flow micro fluidic chamber capable of enriching and isolating viable and/or fixed circulating tumor cells (CTCs) or disseminated tumor cells (DTCs) from a suspension of cells and quantitatively or qualitatively analyzing that subpopulation using molecular analysis methods for diagnosis of tumoral conditions and/or corresponding state of advance of the cancer, whereby the sample can be obtained by minimally invasive methods.

In view of the above, it will be seen that the several advantages of the disclosure are achieved and other advantageous results attained. As various changes could be made in the above devices and methods, it is intended that all matter contained in the above description and shown in the accompanying drawings shall be interpreted as illustrative and not in a limiting sense. The scope of the invention is defined by the appended claims.

When introducing elements of the present disclosure or the various versions, embodiment(s) or aspects thereof, the articles "a", "an", "the" and "said" are intended to mean that there are one or more of the elements. The terms "comprising", "including" and "having" are intended to be inclusive and mean that there may be additional elements other than the listed elements.

## Claims

**1.** A flow chamber (305) for separating target particles from non-target particles in a sample, the flow chamber comprising:

> an exterior surface area and an interior surface area, the interior surface defining a floor and a ceiling;
> a first port (320) for the introduction into the flow chamber of a first fluid (310) with no sample;
> a second port (335) for the introduction into the flow chamber of a sample (325) containing target particles (340) and non-target particles (355);
> a collection port (345) and a waste disposal port (360); and
> an electrode element (200) affixed to a floor area of the flow chamber, wherein the electrode element (200) comprises:

>> a first electrode zone (205) that is associated with a first inter-electrode pitch (245) that extends from a midpoint of one of a plurality of first conductive traces (215) to a midpoint of an adjacent first conductive trace in the first zone, wherein the plurality of first conductive traces (215) have a first conductive trace width (225), the plurality of first conductive traces (215) are spaced apart from each other by a first inter-electrode gap, and the first electrode zone (205) is configured to generate a first electric field when an electrical signal is applied; and
>> a second electrode zone (210) that is associated with a second inter-electrode pitch (250) that extends from a midpoint of one of a plurality of second conductive traces (230) to a midpoint of an adjacent second conductive trace in the second zone, wherein the plurality of second conductive traces (230) have a second conductive trace width (240), the plurality of second conductive traces (230) are spaced apart from each other by a second inter-electrode gap, and the second electrode zone (210) is configured to generate a second electric field with at least one of a different magnitude, a different depth of field, and a different spatial profile than the first electric field when the electrical signal is applied,
>> wherein the electrode element (200) is configured to move the target particles (340) to said collection port (345) and to move the non-target particles (355) to said waste disposal port (360)
>> **characterized in that** the first inter-electrode pitch (245) has a different width than the second inter-electrode pitch (250), and a width of the first inter-electrode gap (220) is the same as a width of the second interelectrode gap (235), the first conductive trace width (225) is different than the second conductive trace width (240), the target particles have diameters of about 10 microns, the widths of the first and second inter-electrode gaps are each 30 microns, the first conductive trace width (225) is 30 microns, the second conductive trace width (240) is 50 microns.

**2.** A method for separating target particles in a fluidic sample containing target particles and non-target particles using dielectrophoresis in the flow chamber of claim 1, the method comprising:

a) continuously introducing the sample containing target particles and non-target particles into the second port of the flow chamber;

b) continuously introducing an elution buffer into the first port of the flow chamber; wherein the sample and the elution buffer form a fluidic suspension in the flow chamber;

c) exposing the fluidic suspension to an electric field in a defined region of the flow chamber, wherein the electric field is produced by the electrode element; and

d) collecting the target particles at the collection port of the flow chamber.

3. The method of claim 2 wherein the elution buffer has a conductivity of from about 5 mS/m to about 250 mS/m.

4. The method of claim 3, wherein the elution buffer has a conductivity of about 30 mS/m.

5. The method of claim 2, wherein (a) the sample is contained in a sample buffer having a conductivity of from about 5 mS/m to about 250 mS/m; or (b) the electric field comprises an AC voltage of from about 2 Vp-p to about 8 Vp-p, and wherein the frequency of applied voltage comprises from about 10 kHz to about 30 MHz.

**Patentansprüche**

1. Flusskammer (305) zum Trennen von Zielpartikeln von Nicht-Zielpartikeln in einer Probe, wobei die Flusskammer Folgendes aufweist:

einen äußeren Flächenbereich und einen inneren Flächenbereich, wobei die innere Fläche einen Boden und eine Decke definiert;

eine erste Öffnung (320) für die Einbringung eines ersten Fluids (310) ohne Probe in die Flusskammer;

eine zweite Öffnung (320) zum Einbringen einer Probe (325), die Zielpartikel (340) und Nicht-Zielpartikel (355) enthält, in die Flusskammer;

eine Sammelöffnung (345) und eine Abfallentsorgungsöffnung (360); und

ein Elektrodenelement (200), das an einem Bodenbereich der Kammer befestigt ist, wobei das Elektrodenelement (200) Folgendes aufweist:

eine erste Elektrodenzone (205), die einem ersten Abstand (245) zwischen den Elektroden zugeordnet ist, der sich von einem Mittelpunkt von einer von mehreren ersten Leiterbahnen (215) zu einem Mittelpunkt von einer benachbarten ersten Leiterbahn in der ersten Zone erstreckt, wobei die mehreren ersten Leiterbahnen (215) eine erste Leiterbahnbreite (225) aufweisen, wobei die mehreren ersten Leiterbahnen (215) durch einen ersten Abstand zwischen den Elektroden voneinander beabstandet sind, und wobei die erste Elektrodenzone (205) so konfiguriert ist, dass sie ein erstes elektrisches Feld erzeugt, wenn ein elektrisches Signal angelegt wird; und

eine zweite Elektrodenzone (210), die einem zweiten Abstand (250) zwischen den Elektroden zugeordnet ist, der sich von einem Mittelpunkt von einer von mehreren zweiten Leiterbahnen (230) zu einem Mittelpunkt von einer benachbarten zweiten Leiterbahn in der zweiten Zone erstreckt, wobei die mehreren zweiten Leiterbahnen (230) eine zweite Leiterbahnbreite (240) aufweisen, wobei die mehreren zweiten Leiterbahnen (230) durch einen zweiten Abstand zwischen den Elektroden voneinander beabstandet sind, und wobei die zweite Elektrodenzone (210) so konfiguriert ist, dass sie ein zweites elektrisches Feld mit mindestens einer anderen Größe, einer anderen Feldtiefe und einem anderen räumlichen Profil als das erste elektrische Feld erzeugt, wenn das elektrische Signal angelegt wird,

wobei das Elektrodenelement (200) so konfiguriert ist, dass es die Zielpartikel (340) zu der Sammelöffnung (345) und die Nicht-Zielpartikel (355) zu der Abfallentsorgungsöffnung (360) bewegt,

**dadurch gekennzeichnet, dass** der erste Abstand (245) zwischen den Elektroden eine andere Breite als der zweite Abstand(250) zwischen den Elektroden hat, und eine Breite des ersten Spalts (220) zwischen den Elektroden die gleiche ist wie eine Breite des zweiten Spalts (235) zwischen den Elektroden, wobei die erste Leiterbahnbreite (225) eine andere ist als die zweite Leiterbahnbreite (240), wobei die Zielpartikel Durchmesser von etwa 10 Mikrometern haben, wobei die Breiten des ersten und des zweiten Spalts zwischen den Elektroden jeweils 30 Mikrometer betragen, wobei die erste Leiterbahnbreite (225) 30 Mikrometer beträgt, und wobei die zweite Leiterbahnbreite (240) 50 Mikrometer beträgt.

2. Verfahren zum Trennen von Zielpartikeln in einer fluiden Probe, die Zielpartikel und Nicht-Zielpartikel enthält, unter Verwendung von Dielektrophorese in der Flusskammer nach Anspruch 1, wobei das Verfahren Folgendes aufweist:

a) kontinuierliches Einbringen der Probe, die Zielpartikel und Nicht-Zielpartikel enthält, in die zweite Öffnung der Flusskammer;
b) kontinuierliches Einführen eines Elutionspuffers in die erste Öffnung der Flusskammer; wobei die Probe und der Elutionspuffer eine fluide Suspension in der Flusskammer bilden;
c) Exponieren der fluiden Suspension an ein elektrisches Feld in einem definierten Bereich der Flusskammer, wobei das elektrische Feld durch das Elektrodenelement erzeugt wird; und
d) Sammeln der Zielpartikel an der Sammelöffnung der Flusskammer.

3. Verfahren nach Anspruch 2, wobei der Elutionspuffer eine Leitfähigkeit von etwa 5 mS/m bis etwa 250 mS/m aufweist.

4. Verfahren nach Anspruch 3, wobei der Elutionspuffer eine Leitfähigkeit von etwa 30 mS/m aufweist.

5. Verfahren nach Anspruch 2, wobei (a) die Probe in einem Probenpuffer mit einer Leitfähigkeit von etwa 5 mS/m bis etwa 250 mS/m enthalten ist; oder (b) das elektrische Feld eine Wechselspannung von etwa 2 Vp-p bis etwa 8 Vp-p aufweist, und wobei die Frequenz der angelegten Spannung von etwa 10 kHz bis etwa 30 MHz reicht.

**Revendications**

1. Chambre d'écoulement (305) pour séparer les particules cibles des particules non cibles dans un échantillon, la chambre d'écoulement comprenant :

une zone de surface extérieure et une zone de surface intérieure, la surface intérieure définissant un plancher et un plafond ;
un premier orifice (320) pour l'introduction dans la chambre d'écoulement d'un premier fluide (310) sans échantillon ;
un deuxième orifice (335) pour l'introduction dans la chambre d'écoulement d'un échantillon (325) contenant des particules cibles (340) et des particules non cibles (355) ;
un orifice de collecte (345) et un orifice d'élimination de déchets (360) ; et
un élément d'électrode (200) fixé à une zone de plancher de la chambre d'écoulement, où l'élément d'électrode (200) comprend :

une première zone d'électrode (205) qui est associée à un premier pas inter-électrodes (245) qui s'étend d'un point médian de l'une d'une pluralité de premières traces conductrices (215) à un point médian d'une première trace conductrice adjacente dans la première zone, où la pluralité de premières traces conductrices (215) ont une première largeur de trace conductrice (225), la pluralité de premières traces conductrices (215) sont espacées les unes des autres par un premier espace interélectrode, et la première zone d'électrode (205) est configurée pour générer un premier champ électrique lorsqu'un signal électrique est appliqué ; et
une deuxième zone d'électrode (210) qui est associée à un deuxième pas inter-électrodes (250) qui s'étend d'un point médian de l'une d'une pluralité de deuxièmes traces conductrices (230) à un point médian d'une deuxième trace conductrice adjacente dans la deuxième zone, où la pluralité de deuxièmes traces conductrices (230) ont une deuxième largeur de trace conductrice (240), la pluralité de deuxièmes traces conductrices (230) sont espacées les unes des autres par un deuxième espace inter-électrodes, et la deuxième zone d'électrode (210) est configurée pour générer un deuxième champ électrique avec au moins une amplitude différente, une profondeur de champ différente et un profil spatial différent du premier champ électrique lorsque le signal électrique est appliqué,
dans laquelle l'élément d'électrode (200) est configuré pour déplacer les particules cibles (340) vers ledit orifice de collecte (345) et pour déplacer les particules non cibles (355) vers ledit orifice d'élimination de déchets (360)

**caractérisée en ce que** le premier pas inter-électrodes (245) a une largeur différente du deuxième pas inter-électrodes (250), et une largeur du premier espace inter-électrodes (220) est identique à une largeur du deuxième espace inter-électrodes (235), la première largeur de trace conductrice (225) est différente de la deuxième largeur de trace conductrice (240), les particules cibles ont des diamètres d'environ 10 microns, les largeurs des premier et deuxième espaces inter-électrodes sont chacune de 30 microns, la première largeur de trace conductrice (225) est de 30 microns, la deuxième largeur de trace conductrice (240) est de 50 microns.

**2.** Procédé de séparation de particules cibles dans un échantillon fluidique contenant des particules cibles et des particules non cibles en utilisant la diélectrophorèse dans la chambre d'écoulement de la revendication 1, le procédé comprenant :

    a) l'introduction en continu de l'échantillon contenant des particules cibles et des particules non cibles dans le deuxième orifice de la chambre d'écoulement ;
    b) l'introduction en continu d'un tampon d'élution dans le premier orifice de la chambre d'écoulement ; où l'échantillon et le tampon d'élution forment une suspension fluidique dans la chambre d'écoulement ;
    c) l'exposition de la suspension fluidique à un champ électrique dans une région définie de la chambre d'écoulement, où le champ électrique est produit par l'élément d'électrode ; et
    d) la collecte des particules cibles au niveau de l'orifice de collecte de la chambre d'écoulement.

**3.** Procédé de la revendication 2, dans lequel le tampon d'élution a une conductivité allant d'environ 5 mS/m à environ 250 mS/m.

**4.** Procédé de la revendication 3, dans lequel le tampon d'élution a une conductivité d'environ 30 mS/m.

**5.** Procédé de la revendication 2, dans lequel (a) l'échantillon est contenu dans un tampon d'échantillon ayant une conductivité allant d'environ 5 mS/m à environ 250 mS/m ; ou (b) le champ électrique comprend une tension alternative allant d'environ 2 Vp-p à environ 8 Vp-p, et dans lequel la fréquence de la tension appliquée comprend d'environ 10 kHz à environ 30 MHz.

FIG. 1

FIG. 2

FIG. 3

FIG. 4

FIG. 5

FIG. 6

FIG. 7

FIG. 8

FIG. 9

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120031759 A1
- WO 2013028573 A

### Non-patent literature cited in the description

- **CHO.** *Mol Cancer,* 2007, vol. 6, 25
- **AVRIL, N. et al.** *The Journal of Nuclear Medicine,* May 2009, vol. 50 (5), 55S-63S
- **GUPTA et al.** *Biomicrofluidics,* 2012, vol. 6, 024133
- **COHEN, S.J. et al.** *J. Clin. Oncol.,* 2008, vol. 26, 3213
- **MAHESWARAN, S. et al.** *N. Engl. J. Med.,* 2008, 359-366
- **DESITTER, I. et al.** *Anticancer Res,* 2011, vol. 31, 427
- **NAGRATH, S. et al.** *Nature,* 2007, vol. 450, 1235
- **BUDD, G.T. ; CRISTOFANILLI, M. ; ELLIS, M.J. ; STOPECK, A. ; BORDEN, E. ; MILLER, M.C. ; MATERA, J. ; REPOLLET, M. ; DOYLE, G.V. ; TERSTAPPEN, L.W.** Circulating tumor cells versus imaging--predicting overall survival in metastatic breast cancer. *Clin Cancer Res,* 2006, vol. 12 (21), 6403-6409
- **DE BONO, J.S. ; SCHER, H.I. ; MONTGOMERY, R.B. ; PARKER, C. ; MILLER, M.C. ; TISSING, H. ; DOYLE, G.V. ; TERSTAPPEN, L.W. ; PIENTA, K.J. ; RAGHAVAN, D.** Circulating tumor cells predict survival benefit from treatment in metastatic castration-resistant prostate cancer. *Clin Cancer Res,* 2008, vol. 14 (19), 6302-6309
- **COHEN, S.J. ; PUNT, C.J. ; IANNOTTI, N. ; SAIDMAN, B.H. ; SABBATH, K.D. ; GABRAIL, N.Y. ; PICUS, J. ; MORSE, M. ; MITCHELL, E. ; MILLER, M.C.** Relationship of circulating tumor cells to tumor response, progression-free survival, and overall survival in patients with metastatic colorectal cancer. *J Clin Oncol,* 2008, vol. 26 (19), 3213-3221
- **CRISTOFANILLI, M. ; BUDD, G.T. ; ELLIS, M.J. ; STOPECK, A. ; MATERA, J. ; MILLER, M.C. ; REUBEN, J.M. ; DOYLE, G.V. ; ALLARD, W.J. ; TERSTAPPEN, L.W.** Circulating tumor cells, disease progression, and survival in metastatic breast cancer. *N Engl J Med,* 2004, vol. 351 (8), 781-791
- **KADAKSHAM et al.** Dielectrophoresis induced by clustering regimes of viable yeast cells. *Electrophoresis,* 2005, vol. 26, 3738-3744
- **SANCHO et al.** Interaction between cells in dielectrophoresis and electrorotation experiments. *Biomicrofuidics,* 2010, vol. 4, 022802